# EUROPÄISCHE PATENTANMELDUNG

(11) **EP 4 201 391 A1**
(43) Veröffentlichungstag der Anmeldung: **28.06.2023**
(21) Anmeldenummer: 21217466.8
(22) Anmeldetag: 23.12.2021
(51) Int. Cl.: A61K 6/818, A61K 6/822, A61K 6/824, C04B 35/486

(54) **MEHRSCHICHTIGER AUFBAU**

(71) Anmelder: VITA-ZAHNFABRIK H. Rauter GmbH & Co. KG, 79713 Bad Säckingen (DE)
(72) Erfinder: GÖDIKER, Michael, 79713 Bad Säckingen (DE); NEUN, Christopher, 79713 Bad Säckingen (DE)
(74) Vertreter: dompatent von Kreisler Selting Werner - Partnerschaft von Patent- und Rechtsanwälten mbB

(57) **Zusammenfassung**

Die vorliegende Erfindung betrifft einen Formkörper mit Farbverlauf zur Verwendung in der Herstellung dentaler Restaurationen erhältlich durch Sintern eines Pressformkörpers umfassend zwei oder mehr farblich unterschiedliche keramische Pulverschichten, wobei jede Pulverschicht mindestens 80 Gew.-% ZrO2 aufweist und im Wesentlichen frei von Eisenoxid ist, dadurch gekennzeichnet, dass wenigstens eine Pulverschicht Terbiumoxid (Tb4O7) aufweist.

## Beschreibung

Die vorliegende Erfindung betrifft einen gesinterten Formkörper mit Farbverlauf zur Verwendung in der Herstellung dentaler Restaurationen erhältlich durch Sintern eines Pressformkörpers umfassend 2 oder mehr farblich unterschiedliche keramische Pulverschichten sowie ein Verfahren zu dessen Herstellung als auch die Verwendung der Formkörpers zur Herstellung dentaler Restaurationen.

Zirkoniumdioxid-Keramiken haben aufgrund ihrer Härte und guten Bearbeitbarkeit sowie aufgrund ihrer steuerbaren Transluzenzeigenschaften Einzug in der Dentaltechnik für die Herstellung dentaler Restaurationen gehalten. Die Einfärbung der ZrO2-Keramiken erfolgt üblicherweise durch Zusatz von färbenden Oxiden, die zusammen mit dem Zirkoniumdioxid versintert werden. Um einen der natürlichen Zahnfarbe angepassten Verlauf der Farbe zu erhalten, bietet es sich an, die Konzentration der färbenden Oxide schichtweise zu verändern und so einzustellen, dass der Farbverlauf der natürlichen Zahnfarbe möglichst nahe kommt. Problematisch ist hierbei jedoch, dass die färbenden Metalloxide einen Einfluss auf das Sinterverhalten der Zirkoniumdioxide-Keramiken haben können. Dies wurde insbesondere beim Einsatz von Eisenoxiden festgestellt. Ein schichtweiser Aufbau einer Zirkoniumdioxid-Keramik, bei dem die Eisenoxidkonzentration schichtweise geändert ist, führt zu einem Sinterverzug der Keramik. Dieser Verzug muss anschließend aufwändig ausgeglichen/korrigiert werden. Desweiteren führt ein schichtweise unterschiedliches Sinterverhalten auch zu unterschiedlichen Härten der einzelnen Schichten, was die Bearbeitung mit subtraktiven Mitteln zur Herstellung von dentalen Restaurationen zusätzlich erschwert.

Der vorliegenden Erfindung liegt daher die Aufgabe zugrunde einen gesinterten Formkörper zur Verfügung zu stellen, der die vorgenannten Probleme löst, insbesondere verzugsfrei über einen großen Temperaturbereich gesintert werden kann.

Darüber hinaus besteht eine Aufgabe der vorliegenden Erfindung darin einen keramischen gesinterten Formkörper mit Farbverlauf bereitzustellen, der eine weitestgehend homogene Härte aufweist und damit gut bearbeitbar ist.

Gelöst werden die zuvor genannten Aufgaben durch einen gesinterten Formkörper gemäße Anspruch 1 der vorliegenden Erfindung.

Gegenstand der vorliegenden Erfindung in einer ersten Ausgestaltung ist ein gesinterter Formkörper mit Farbverlauf zur Verwendung in der Herstellung dentaler Restaurationen erhältlich durch Sintern eines Pressformkörpers umfassend 2 oder mehr farblich unterschiedliche keramische Pulverschichten, wobei jede Pulverschicht mindestens 80 Gew.-% ZrO₂ aufweist und im Wesentlichen frei von Eisenoxid ist, dadurch gekennzeichnet, dass wenigstens eine Pulverschicht Terbiumoxid aufweist.

Im Wesentlichen frei von Eisenoxid im Sinne der vorliegenden Erfindung bedeutet, dass weniger als 0,01 Gew.-%, vorzugsweise weniger als 0.001 Gew.% oder weniger als 0,0001 Gew.-%, jeweils bezogen auf das Gesamtgewicht der Pulverschicht, vorliegt. In einer bevorzugten Ausgestaltung ist der gesinterte Formkörper frei von Eisenoxid.

Der Einsatz von Terbiumoxid hat sich als hervorragend für die Einfärbung als auch für die Verzugsstabilität herausgestellt.

Terbiumoxid im Sinne der vorliegenden Erfindung umfasst grundsätzlich alle Oxide des Terbiums. Bevorzugt ist das Terbiumoxid ausgewählt aus der Gruppe bestehend aus Tb₂O₃, Tb₄O₇, Tb₇O₁₂, Tb₁₁O₂₀, TbO₂ und Mischung hiervon.

Besonders bevorzugt eingesetzt werden kann Terbiumoxid ausgewählt aus der Gruppe bestehend aus Tb₄O₇, Tb₇O₁₂, Tb₁₁O₂₀ und beliebige Mischungen hiervon.

Speziell bevorzugt liegt das Terbiumoxid als Tb₄O₇ oder als Mischung von Tb₄O₇ mit Tb₇O₁₂, und/oder Tb₁₁O₂₀ vor.

Bevorzugt sind zudem Terbiumoxide, die mit gemischter Valenz vorliegen.

In einer bevorzugten Ausführungsform umfasst wenigstens eine Pulverschicht, bevorzugt wenigstens 2 Pulverschichten oder wenigstens 3 Pulverschichten oder wenigstens 4 Pulverschichten, speziell 5 Pulverschichten oder jede Pulverschicht Terbiumoxid in einer Menge von 0,001 bis 0,15 Gew.-%, vorzugsweise 0,01 bis 0,1 Gew.-%, insbesondere 0,02 bis 0,06 Gew.-% Terbiumoxid.

Es hat sich überraschend gezeigt, dass jede der gesinterten Pulverschichten eine im Wesentlichen gleiche Volumenveränderung über einen Temperaturbereich von 25 bis 1600 °C, insbesondere über einen Temperaturbereich von 50 bis 1400 °C oder 900 bis 1400 °C und speziell über einen Temperaturbereich von 900 bis 1350 °C, erfährt.

Im Sinne der vorliegenden Erfindung bedeutet eine im Wesentlichen gleiche Volumenveränderung, dass die Differenz der Volumenveränderung bei einer vorgegebenen Temperatur im Bereich von 25 bis 1600 °C von 2 Schichten des erfindungsgemäßen gesinterten Rohlings maximal 1%, vorzugsweise maximal 0.5 %, insbesondere maximal 0.05 %, beträgt.

Dies führt für die erfindungsgemäßen gesinterten Formkörper zu einem geringen Sinterverzug.

In einer weiteren bevorzugten Ausgestaltung der vorliegenden Erfindung weist jede Pulverschicht eine unterschiedliche Konzentration Terbiumoxid, Erbiumoxid und Cobaltoxid auf.

In einer bevorzugten Ausgestaltung weist jede Pulverschicht ausschließlich Terbiumoxid, Erbiumoxid und Cobaltoxid als färbende Metalloxide auf.

In einer weiteren Ausgestaltung weist jede Pulverschicht Terbiumoxid, Erbiumoxid und Cobaltoxid auf ist aber im Wesentlichen frei von weiteren färbenden Metalloxiden.

Im Wesentlichen frei von weiteren färbenden Metalloxiden im Sinne der vorliegenden Erfindung bedeutet, dass weniger als 0,01 Gew.-%, vorzugsweise weniger als 0.001 Gew.-% oder weniger als 0,0001 Gew.-%, jeweils bezogen auf das Gesamtgewicht der Pulverschicht, vorliegt. In einer bevorzugten Ausgestaltung ist der gesinterte Formkörper frei von weiteren färbenden Metalloxiden.

Bevorzugt weist wenigstens eine Pulverschicht, besonders bevorzugt 2 oder mehr Pulverschichten, insbesondere jede Pulverschicht Erbiumoxid (Er₂O₃) in einer Menge von 0,1 bis 1,0 Gew.-%, speziell 0,2 bis 0,8 Gew.-%, jeweils bezogen auf das Gesamtgewicht der Pulverschichtzusammensetzung, auf. Es hat sich überraschend gezeigt, dass Erbiumoxid in den Mengen als färbendes Metalloxid eingesetzt werden kann, ohne die Sintereigenschaften negativ zu beeinflussen. In einer weiteren bevorzugten Ausgestaltung der vorliegenden Erfindung ist das Gewichtsverhältnis von Terbiumoxid zu Cobaltoxid in wenigstens einer Pulverschicht, vorzugsweise in wenigstens 2 oder 3 Pulverschichten, insbesondere in jeder Pulverschicht im Bereich von 80:1 zu 5:1, vorzugsweise 75:1 bis 10:1 und speziell 60:1 bis 15:1. Insbesondere gute Farb- und Sintereigenschaften konnten mit den vorgenannten eingestellten Gewichtsverhältnissen erzielt werden speziell bei Einsatz von Tb₄O₇.

Jede Pulverschicht kann bevorzugt durch Vermischen von Basispulvern hergestellt werden. In einer bevorzugten Ausgestaltung erfolgt die Herstellung jeder Pulverschicht durch Vermischen von 2 oder mehr, vorzugsweise 3 oder mehr, insbesondere durch 4 oder mehr Basispulver. Das Verwenden einer begrenzten Menge von Basispulvern in unterschiedlichen Mengen für die Herstellung jeder Pulverschicht hat Vorteile hinsichtlich der Kompatibilität der einzelnen übereinander angeordneten Schichten und für deren Farbverlauf im gesinterten Formkörper. Geeignete Basispulver können beispielsweise von der Firma Tosoh bezogen werden.

Die gesinterten Formkörper der vorliegenden Erfindung weisen bevorzugt einen graduellen Farbverlauf auf. In einer Ausgestaltung der Erfindung weist der Rohling farblich unterschiedliche Schichten auf und durch den Sintervorgang entsteht ein gradueller Farbverlauf.

In einer bevorzugten Ausführungsform der vorliegenden Erfindung weisen wenigstens 2, vorzugsweise alle Pulverschichten einen im Wesentlichen identischen Yttriumoxid Anteil auf.

Im Wesentlichen identischer Yttriumoxid-Anteil in 2 oder mehreren Schichten im Sinne der vorliegenden Erfindung bedeutet, dass die Differenz innerhalb der Schichten nicht mehr als 0,1 Mol-%, vorzugsweise nicht mehr als 0,05 Mol-%, insbesondere weniger als 0,01 Mol-% ausmacht.

In einer alternativen Ausgestaltung der vorliegenden Erfindung weist der gesinterte Formkörper einen Transluzenzverlauf auf. Vorzugsweise zeigt der erfindungsgemäße Formkörper eine schichtweise Zunahme der Yttriumoxidmenge auf.

In einer bevorzugten Ausführungsform weisen die erfindungsgemäßen Formkörper Pulverschichten auf mit jeweils mindestens 0,02 Gew.-% Al₂O₃.

Es hat sich als vorteilhaft herausgestellt, dass die Pulverschichten jeweils mindestens 0,02 Gew.-% Al₂O₃, vorzugsweise Al₂O₃ in einer Menge von 0,03 bis 0,15 Gew.-%, insbesondere Al₂O₃ in einer Menge von 0,03 bis 0,08 Gew.-% aufweisen. Die bevorzugte Menge Al₂O₃ in den einzelnen Pulverschichten führt zu einem verbesserten Stabilitäts- und Festigkeitsverhalten der erfindungsgemäßen Formkörper.

In einem weiteren Aspekt der vorliegenden Erfindung weisen wenigstens eine, vorzugsweise wenigstens 2 oder wenigstens 3 der Pulverschichten, insbesondere alle Pulverschichten Y₂O₃ und/oder Er₂O₃, vorzugsweise in einer Menge von wenigstens 3 Gew.-%, insbesondere von wenigstens 5 Gew.-% oder wenigstens 6 Gew.-% und insbesondere von 4,5 bis 12 Gew.-%, speziell von 6 bis 10 Gew.% bezogen auf das Gesamtgewicht der Bestandteile der Pulverschicht, auf.

Das Y₂O₃ hat eine die Zirkoniumdioxidkristallphasen stabilisierende Funktion und ist kein färbendes Metalloxid im Sinne der vorliegenden Erfindung.

Das Erbiumoxid (Er₂O₃) hat eine stabilisierende Funktion für die ZrO2-Kristallphasen und ist ein färbendes Metalloxid im Sinne der vorliegenden Erfindung.

Bevorzugt weisen die Pulverschichten Zirkondioxid und/oder HfO₂ in einer Menge von wenigstens 89 Gew.-%, vorzugsweise in einer Menge von 89 bis 98 Gew.-%, insbesondere von 90 bis 96 Gew.-%, jeweils bezogen auf das Gesamtgewicht der Bestandteile der Pulverschicht, auf.

In einer bevorzugten Ausgestaltung umfassen die Pulverschichten Zirkoniumdioxid und Hafniumdioxid, besonders bevorzugt beträgt die Menge an Hafniumdioxid 0,1 bis 5 Gew.-%, speziell 0,5 bis 2,5 Gew.-%, bezogen auf das Gesamtgewicht von Zirkoniumdioxid und Hafniumdioxid.

In einer bevorzugten Ausgestaltung der vorliegenden Erfindung besteht der Pressformkörper aus 3 oder 4 oder insbesondere aus 5 verschiedenen keramischen Pulverschichten.

In einer weiteren bevorzugten Ausgestaltung der vorliegenden Erfindung besteht der Pressformkörper aus 5 keramischen Pulverschichten, wobei die erste Pulverschicht 20 bis 30 %, vorzugsweise 22 bis 28 %, die zweite Pulverschicht 10 bis 20 %, vorzugsweise 12 bis 18 %, die dritte Pulverschicht 15 bis 25 %, vorzugsweise 17 bis 23 %, die vierte Pulverschicht 10 bis 20 %, vorzugsweise 12 bis 18 % und die fünfte Pulverschicht 20 bis 30 %, vorzugsweise 22 bis 28 %, der Gesamtdicke der übereinander angeordneten Pulverschichten ausmacht und mit der Maßgabe, dass sich die Gesamtdicke auf 100 % ergänzt.

In einer Ausgestaltung der Erfindung wird der gesinterte Formkörper zunächst vorgesintert und durch subtraktive Verfahren bearbeitet und vorzugsweise anschließend in einem weiteren Schritt endgesintert wird.

Die gesinterten Formkörper der vorliegenden Erfindung finden insbesondere Verwendung als dentale Restaurationen oder für die Herstellung dentaler Restaurationen.

In einer weiteren Ausgestaltung der vorliegenden Erfindung weist wenigstens eine Pulverschicht, vorzugsweise alle Pulverschichten zusätzlich organische Bestandteile, vorzugsweise in einer Menge von 3 - 6 Gew.-%, insbesondere in einer Menge von 4 - 5 Gew.-% auf. Als organische Bestandteile kommen insbesondere Bindemittel und Presshilfsmittel in Frage, die im Entbinderungsschritt thermisch leicht entfernt werden können. Geeignete Binder für Zirkoniumdioxid-Sinterpulver sind dem Fachmann bekannt. Hierzu zählt beispielsweise Polyvinylalkohol (PVA).

Bevorzugt weisen die Schichtpulver eine Schüttdichte unterhalb von 1,2 g/cm³ auf.

Es hat sich als vorteilhaft herausgestellt, Schichtpulver einzusetzen, die eine durchschnittliche Granulatgröße D₅₀ von 35 µm - 85 µm, bevorzugt von 40 µm - 80 µm und insbesondere von 50 µm - 70 µm oder 40 - 60 µm aufweisen. Gemessen werden die Granulatpulver trocken mittels Laserbeugung mittels eines Cilas Granulometer.

Üblicherweise weisen die anorganischen Bestandteile der Basispulver, also nach Entfernung der organischen Bestandteile, wie Bindemittel, etc., eine Partikelgröße D₅₀ von 0,1 bis 1 µm, bevorzugt von 0,2 µm - 0.8 µm und insbesondere von 0,2 µm - 0,7 µm, gemessen mittels Laserbeugung, auf. Es wurde gefunden, dass die Partikelgrößen einen positiven Beitrag zur Sinterung und insbesondere für die Farbübergänge zwischen den einzelnen Pulverschichten sorgen.

Der erfindungsgemäß zu sinternde Pressformkörper kann durch schichtweises übereinander Anordnen von 2 oder 3 oder insbesondere 4 oder fünf oder mehr keramischen Pulverschichten erhalten werden. Die Anordnung der Schichten kann beispielsweise in einem zylindrischen Behälter erfolgen unter Ausbildung von Scheiben oder Disks. Üblicherweise kann nach jedem Schichtauftrag ein uniaxiales Verpressen der Pulverschichten erfolgen. Dies kann beispielsweise durch einen Pressstempel erfolgen, wobei jedoch lediglich eine Vorverfestigung erfolgt. Das uniaxiale Verpressen der Schichten senkrecht zur Schichtoberfläche erfolgt bevorzugt bei einem Druck von 10-20 MPa, insbesondere 12-15 MPa.

In einer weiteren bevorzugten Ausgestaltung erfolgt das Verpressen der schichtweise übereinander angeordneten keramischen Pulverschichten zur Ausbildung eines Pressformkörpers durch Verpressen zunächst uniaxial und senkrecht zur Schichtoberfläche, vorzugsweise unter Ausbildung eines vorverdichteten Pressformkörpers mit einer Dichte unterhalb 2,8 g/cm³, vorzugsweise mit einer Dichte im Bereich von 2,5 - 2,7 g/cm³, bspw. 2,65 g/cm³. Das uniaxiale Vorverdichten kann zu einer besseren und innigeren Vermischung und damit gleichmäßigem Übergang zwischen den Schichten führen.

In einer weiteren bevorzugten Ausgestaltung erfolgt das Verpressen zur Herstellung des Pressformkörpers isostatisch, wobei das isostatische Verpressen vorzugsweise im Anschluss eines uniaxialen Vorverdichtens erfolgt, unter Ausbildung eines Pressformkörpers mit einer Dichte unterhalb von 3,4 g/cm³, insbesondere einer Dichte von 2,80 - 3,3 g/cm³, speziell mit einer Dichte von 2,85 - 3,25 g/cm³. Das isostatische Verpressen erfolgt bevorzugt, nachdem alle Schichten des Formpresskörpers angeordnet sind. Geeignete Drucke für das isostatische Verpressen liegen dabei gewöhnlich im Bereich von 500 - 10000 bar, bevorzugt im Bereich von 800 - 8000 bar, bspw. 1000 - 7000 bar oder 1000 - 3000 bar.

Die Dicken der einzelnen Pulverschichten des Pressformkörpers können variieren. In einer bevorzugten Ausgestaltung unterscheiden sich wenigstens zwei der keramischen Pulverschichten hinsichtlich ihrer Dicke. Vorzugsweise weisen wenigstens zwei der keramischen Pulverschichten des Pressformkörpers einen Dickenunterschied von wenigstens 5% auf. Typischerweise können die Pressformkörper in Form zylindrischer, kreisförmiger Scheiben mit Durchmessern im Bereich von 50 bis 200 mm, beispielsweise 75 bis 150 mm vorliegen. Die Gesamtdicke der zylindrischen Scheiben kann dabei beispielsweise im Bereich von 8 bis 40 mm, vorzugsweise von 10 bis 30 mm, speziell von 13 bis 25 mm liegen. Die Maße beziehen sich dabei auf den Pressformkörper im ungesinterten Zustand.

Hinsichtlich der Farbgestaltung und der anschließenden Bearbeitung hat es sich als vorteilhaft herausgestellt, dass wenigstens eine der äußeren keramischen Pulverschichten, vorzugsweise beide äußeren keramischen Pulverschichten des Pressformkörpers eine größere Dicke aufweist/aufweisen als eine zwischen den äußeren keramischen Pulverschichten liegende keramische Pulverschicht. Insbesondere bei der Verwendung der erfindungsgemäß hergestellten keramischen Formkörper für die Herstellung dentaler Restaurationen hat sich der zuvor beschriebene Schichtaufbau mit wenigstens einer dickeren äußeren Schicht als vorteilhaft erwiesen, da dies für die Bearbeitung in CAD/CAM Systemen oder anderen subtraktiven Bearbeitungsverfahren einen geeigneten Aufbau darstellt.

In einer speziell bevorzugten Ausgestaltung der vorliegenden Erfindung umfasst der Pressformkörper fünf keramische Pulverschichten, wobei die erste Pulverschicht 20 - 30%, vorzugsweise 22 - 28%, die zweite Pulverschicht 10 - 20%, vorzugsweise 12 - 18%, die dritte Pulverschicht 15 - 25%, vorzugsweise 17 - 23%, die vierte Pulverschicht 10 - 20%, vorzugsweise 12 - 18% und die fünfte Pulverschicht 20 - 30%, vorzugsweise 22 - 28% der Gesamtdicke der übereinander angeordneten Pulverschichten ausmacht und mit der Maßgabe, dass sich die Gesamtdicke auf 100% ergänzt.

In einer weiteren Ausgestaltung der vorliegenden Erfindung erfolgt die Sinterung bei einer Temperatur im Bereich von 950 - 1100°C, vorzugsweise von 980 - 1050°C unter Ausbildung eines vorgesinterten keramischen Formkörpers (Weißling). Üblicherweise erfolgt die Sinterung über einen Zeitraum, der ausreicht, um die vorhandenen Bindemittel zu entfernen und dem Pressformkörper eine genügende Festigkeit für die Bearbeitung durch subtraktive Verfahren zu verleihen. Die vorgesinterten und entbinderten Pressformkörper werden als Weißlinge bezeichnet.

In einer Ausgestaltung erfolgt die Sinterung zur Herstellung des Weißlings über einen Zeitraum oberhalb von 30 Minuten, bevorzugt oberhalb von 1 Stunde, insbesondere oberhalb von 20 Stunden oder oberhalb von 50 Stunden, beispielsweise 60 bis 200 Stunden oder 70 bis 150 Stunden.

Insbesondere zur Herstellung keramischer dentaler Restaurationen bietet es sich an, dass der vorgesinterte keramische Formkörper durch subtraktive Verfahren bearbeitet wird und vorzugsweise anschließend in einem weiteren Schritt endgesintert wird. Bei Anwendung subtraktiver Verfahren wird üblicherweise die Sinterschwindung mit einberechnet.

Die Endsinterung erfolgt üblicherweise bei Temperaturen oberhalb von 1350 °C, bevorzugt oberhalb von 1400 °C, speziell im Bereich von 1420 °C bis 1600 °C oder 1450 °C bis 1590°C oder 1480 bis 1580°C.

Die Sinterdauer für die Endsinterung erfolgt üblicherweise über einen Zeitraum von mehr als 4 Minuten, bevorzugt mehr als 5 Minuten, insbesondere im Bereich von 5 bis 120 Minuten.

Die erfindungsgemäßen Formkörper können insbesondere im Dentalbereich eingesetzt werden. Hier zeichnen sie sich durch eine hohe Kantenfestigkeit bei dentalen Restaurationen, ein hervorragendes Gefüge und eine hohe 3-Punktbiegefestigkeit aus. Die keramischen Formkörper der vorliegenden Erfindung sind daher bevorzugt dentale Restaurationen, wie beispielsweise Inlays, Onlays, Kronen, Brücken oder Veneers.

Ein weiterer Gegenstand der vorliegenden Erfindung ist die Verwendung des erfindungsgemäßen keramischen Formkörpers für dentale Restaurationen oder für die Herstellung dentaler Restaurationen.

Ein weiterer Gegenstand der vorliegenden Erfindung ist die Verwendung eines Basispulvers umfassend Zirkoniumdioxid und Terbiumoxid, insbesondere Tb₄O₇, und Yttriumoxid zur Herstellung von sinterverzugsfreien keramischen dentalen Restaurationen, vorzugsweise mit Farbverlauf.

Das Basispulver umfasst bevorzugt Yttriumoxid in eine Menge von 5 bis 8 Gew.%, vorzugsweise 6 bis 7 Gew.-%, Terbiumoxid in einer Menge von 0,1 bis 0,4 Gew.-%, vorzugsweise 0,1 bis 0,3 Gew.-% und Zirkoniumdioxid in einer Menge oberhalb von 80 Gew.-%, vorzugsweise von 82 Gew.-% bis 94 Gew.-%, speziell 84 Gew.-% bis 90 Gew.-%, jeweils bezogen auf das Gesamtgewicht des Basispulvers.

Ein weiterer Gegenstand der vorliegenden Erfindung ist ein Verfahren zur Herstellung des erfindungsgemäßen gesinterten Formkörpers mit Farbverlauf umfassend die folgenden Schritte:
a) Bereitstellen von 2 oder 3 oder 4 oder 5 oder mehr keramischen Pulverschichten, die schichtweise übereinander angeordnet sind,
b) Verpressen der schichtweise übereinander angeordneten keramischen Pulverschichten zur Ausbildung eines Pressformkörpers und
c) Sintern des in Schritt b) erhaltenen Formkörpers zur Ausbildung eines keramischen Formkörpers, wobei die keramischen Pulverschichten jeweils eine voneinander verschiedene Zusammensetzung aufweisen und wobei jede keramische Pulverschicht eine Mischung aus mindestens zwei verschiedenen Basispulvern umfasst und die Basispulver jeweils mindestens 80 Gew.-% ZrO₂ aufweisen, wobei die Gewichtsangabe bezogen ist auf das Gesamtgewicht des Basispulvers.

Bevorzugte Ausgestaltung des erfindungsgemäßen Verfahrens wurden bereits obenstehend erläutert.

Ein weiterer Gegenstand der vorliegenden Erfindung ist ein gesinterter Formkörper mit Schichtaufbau und Farbverlauf zur Verwendung in der Herstellung dentaler Restaurationen, wobei der Formkörper mindestens drei voneinander unterschiedliche keramische Pulverschichten aufweist und jede Schicht aus mindestens drei oder vier voneinander unterschiedlichen Basispulvern besteht, wobei jedes Basispulver mindestens 80 Gew.-% keramische Oxide aufweist, wobei die Gewichtsangaben jeweils bezogen sind auf das Gesamtgewicht des Basispulvers.

Bevorzugt weisen die keramischen Pulverschichten keramische Oxide auf wie vorangehend definiert. Die einzusetzenden Basispulver entsprechen jeweils den vorangehend definierten Basispulvern.

### Beispiele:

Tabelle 1 zeigt 5 Basispulver A bis E, die für die Zusammensetzungen der keramischen Pulverschichten zum Einsatz kommen. Die Granulatgröße D₅₀ der Basispulver liegt im Bereich von 40-80 µm. Die anorganischen Bestandteile der Basispulver weisen eine Partikelgröße D₅₀ von 0,2 bis 0,7 µm auf.

Die Gewichtsangaben beziehen sich jeweils auf das Gesamtgewicht der Pulverzusammensetzung.

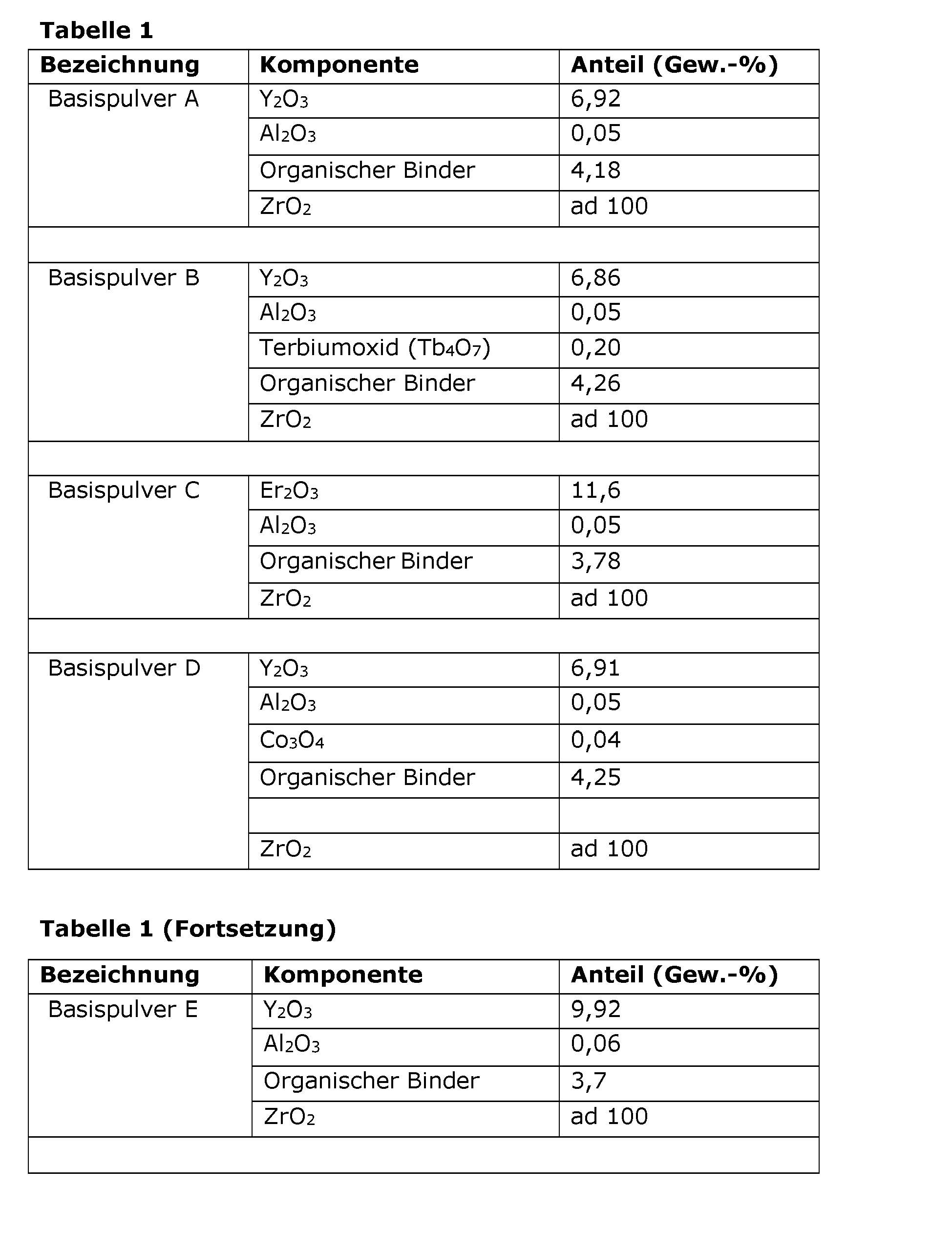

Die in der folgenden Tabelle 2 aufgeführten Anordnungen der Schichten zeigen die Zusammensetzung jeder einzelnen keramischen Pulverschicht in dem Pressformkörper. Die Pressformkörper sind für die Verwendung in der Herstellung dentaler Restaurationen vorgesehen, so dass die Schichtzusammensetzungen entsprechend der Position im Zahn ausgestaltet sind. Die Zusammensetzungen der Pulverschichten werden aus den Basispulvern gebildet, in dem die Anteile variiert werden, um einen idealen Farbverlauf zu erhalten. Die Zusammensetzung jeder Pulverschicht wird durch homogenes Vermischen der Basispulver in den angegebenen Mengen erzielt. Anschließend werden die Pulver schichtweise in eine zylinderförmige Pressform mit einem Durchmesser von 100 mm gegeben und eine Schichtdicke von 18 mm eingestellt. Die Pulverschichten werden uniaxial bei einem Druck von 13 MPa senkrecht zur Schichtoberfläche vorgepresst und anschließend isostatisch bei einem Druck von 2000 bar verpresst.

Nachfolgend erfolgt die Entbinderung bei ca. 1000 °C über einen Zeitraum von ca. 100 Stunden. Die so erhaltenen Weißlinge werden über CAD/CAM Systeme zu dentalen Restaurationen gefräst.

Diese vorgesinterten und bearbeiteten Weißlinge werden anschließend bei 1450 °C über einen Zeitraum von 120 Minuten endgesintert.

**Tabelle 2**

| **Pulverschicht** | Bereich der Restauration | Basispulver A (Gew.-%) | Basispulver B (Gew.-%) | Basispulver C (Gew.-%) | Basispulver D (Gew.-%) |
|---|---|---|---|---|---|
| 1 | Schneide | 56,9 | 28,4 | 4,2 | 10,5 |
| 2 | Dentin/Schneide | 46,9 | 36,5 | 4,7 | 11,9 |
| 3 | Dentin | 43,9 | 41,3 | 5,2 | 9,6 |
| 4 | Dentin/Hals | 36,2 | 47,2 | 5,5 | 11,1 |
| 5 | Hals | 28,8 | 52,9 | 5,8 | 12,5 |

Die keramischen Pulverschichten sind im vorliegenden Beispiel so angeordnet, dass Schicht 1 (Schneide) 25%, Schicht 2 (Dentin/Schneide) 15%, Schicht 3 (Dentin) 20%, Schicht 4 (Dentin/Hals) 15% und Schicht 5 (Hals) 25% der Gesamtdicke des Pressformkörpers ausmacht.

Es hat sich überraschend gezeigt, dass im Vergleich zu Eisenoxid-haltigen Rezepturen, welche zu einer gleichen Farbgebung führen, kein Sinterverzug auch bei höheren Temperaturen beobachtet werden konnte. Im Vergleich dazu wurde bei im Stand der Technik bekannten Eisenoxid-haltigen Rezepturen ein Sinterverzug festgestellt. Die Versinterung nimmt dabei mit zunehmendem Eisenoxid-Anteil zu, so dass auch die Vickershärte im Weißling von hellen Schichten (geringerer Eisenoxid-Anteil) zu dunkleren Schichten (höherer Eisenoxid-Anteil) zunimmt. Im Gegensatz dazu sind die erfindungsgemäßen Ausführungsformen, die mit Terbiumoxid gefärbt werden, über alle Schichten hinweg verzugsfrei und damit ist auch der Grad der Versinterung über alle Schichten hinweg homogen, was wiederum zu einer homogenen Verteilung der Vickershärte führt und damit ebenso zu gleichbleibenden Bearbeitungseigenschaften bei der CAM-Bearbeitung.

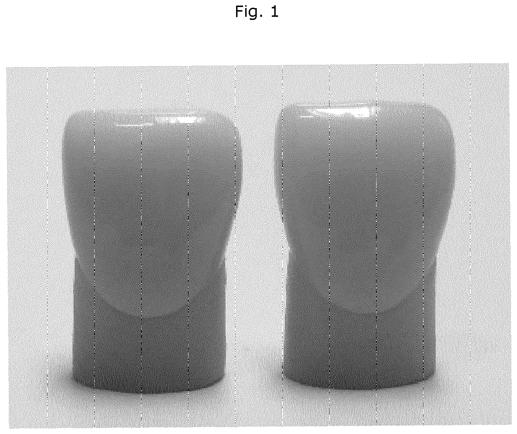

Figur 1 zeigt beispielhaft dentale Restaurationen, die aus dem beispielhaften keramischen Formkörper erhalten werden. Gezeigt sind 2 Frontzahnkronen.

Die Schichtübergänge und Farbübergänge sind fließend. Die Restaurationen weisen eine hervorragende Kantenfestigkeit und Stabilität auf. Ein Nacharbeiten und Nachjustieren der Zahnfarbe ist nicht erforderlich.

Der optimale Aufbau und die Zusammensetzung der Schichten zeigt eine über alle Schichten hinweg weitestgehend homogene Schwindung während der Sinterung. Dies ist insbesondere für eine passgenaue Anfertigung der dentalen Restaurationen von Vorteil, da aufwendiges Nacharbeiten weitestgehend vermieden werden kann.

Die nachfolgende Tabelle 3 zeigt beispielhaft unterschiedliche VITA classical A1-D4^{®} - Zahnfarben, die mit den Basispulvern erstellt werden können.

**Tabelle 3:**

| **Farbe** | **Rohstoff** | **Rezeptur [wt%]** | **Komponente** | **[wt%]** |
|---|---|---|---|---|
| A1 | Basispulver A | 84,62 | Y₂O₃ | 6,71459 |
| | Basispulver B | 10,07 | Al₂O₃ | 0,04985 |
| | Basispulver C | 3,00 | Tb₄O₇ | 0,02193 |
| | Basispulver D | 2,31 | Co₃O₄ | 0,00092 |
| | | | Er₂O₃ | 0,34800 |
| | | | Gesamt: | **7,13530** |
| | | | | |

| **Farbe** | **Rohstoff** | **Rezeptur [wt%]** | **Komponente** | **[wt%]** |
|---|---|---|---|---|
| A2 | Basispulver A | 78,10 | Y₂O₃ | 6,57312 |
| | Basispulver B | 14,00 | Al₂O₃ | 0,04975 |
| | Basispulver C | 5,00 | Tb₄O₇ | 0,02972 |
| | Basispulver D | 2,90 | Co₃O₄ | 0,00116 |
| | | | Er₂O₃ | 0,5800 |
| | | | **Gesamt:** | **7,23375** |
| | | | | |

| **Farbe** | **Rohstoff** | **Rezeptur [wt%]** | **Komponente** | **[wt%]** |
|---|---|---|---|---|
| A3 | Basispulver A | 70,17 | Y₂O₃ | 6,55361 |
| | Basispulver B | 19,44 | Al₂O₃ | 0,04974 |
| | Basispulver C | 5,22 | Tb₄O₇ | 0,04049 |
| | Basispulver D | 5,17 | Co₃O₄ | 0,00207 |
| | | | Er₂O₃ | 0,60552 |
| | | | **Gesamt:** | **7,25143** |
| | | | | |

| **Farbe** | **Rohstoff** | **Rezeptur [wt%]** | **Komponente** | **[wt%]** |
|---|---|---|---|---|
| A3,5 | Basispulver A | 56,28 | Y₂O₃ | 6,43698 |
| | Basispulver B | 28,80 | Al₂O₃ | 0,04966 |
| | Basispulver C | 6,80 | Tb₄O₇ | 0,05902 |
| | Basispulver D | 8,12 | Co₃O₄ | 0,00325 |
| | | | Er₂O₃ | 0,78880 |
| | | | **Gesamt:** | **7,33771** |
| | | | | |

| **Farbe** | **Rohstoff** | **Rezeptur [wt%]** | **Komponente** | **[wt%]** |
|---|---|---|---|---|
| A4 | Basispulver A | 49,74 | Y₂O₃ | 6,44178 |
| | Basispulver B | 30,40 | Al₂O₃ | 0,04967 |
| | Basispulver C | 6,70 | Tb₄O₇ | 0,06219 |
| | Basispulver D | 13,16 | Co₃O₄ | 0,00526 |
| | | | Er₂O₃ | 0,77720 |
| | | | **Gesamt:** | **7,33610** |

Hinsichtlich der Komponenten werden die aufgeführten Gewichtsmengen durch die Binder und das Zirkoniumdioxid auf 100 Gew.-% aufgefüllt.

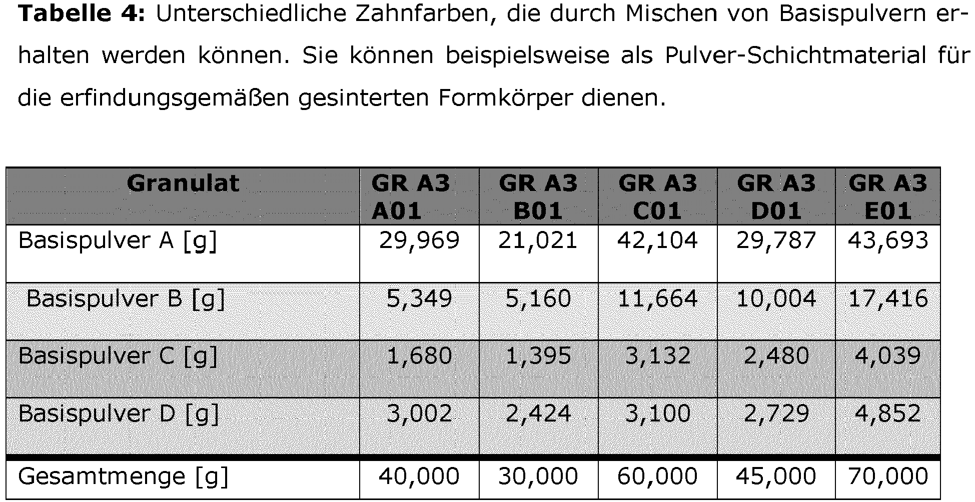

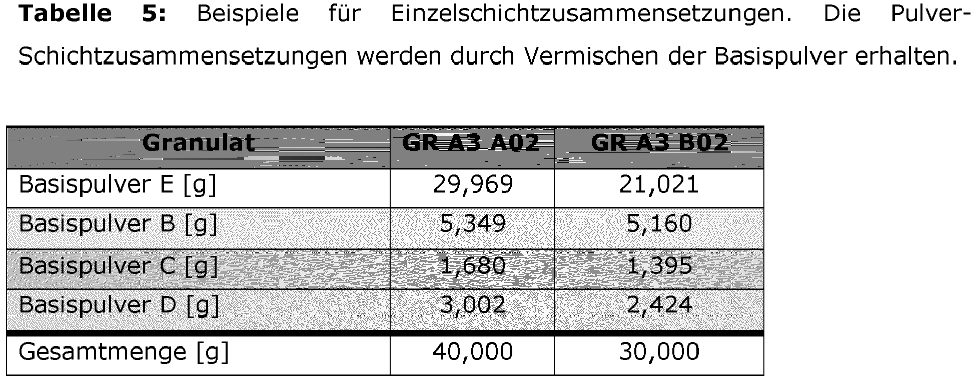

Die Pulverzusammensetzungen für die einzelnen Pulverschichten des erfindungsgemäßen gesinterten Formkörpers werden durch Vermischen der Basispulver erhalten.

Zur Herstellung des mehrschichtigen gesinterten Formkörpers werden die in den Tabellen 4 und 5 aufgeführten Schichtpulverzusammensetzungen in einer Weber Presse (Programm Nr. 22; 100 MPa = 80 kN) verpresst. Die Gesamt-Einwaage pro Block beträgt 40g. Die unterschiedlichen Schichten teilen sich in folgender Weise wie in den Tabellen 6 und 7 dargestellt auf:

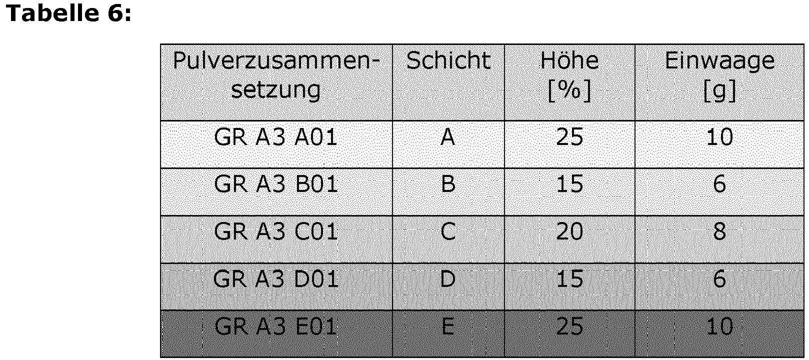

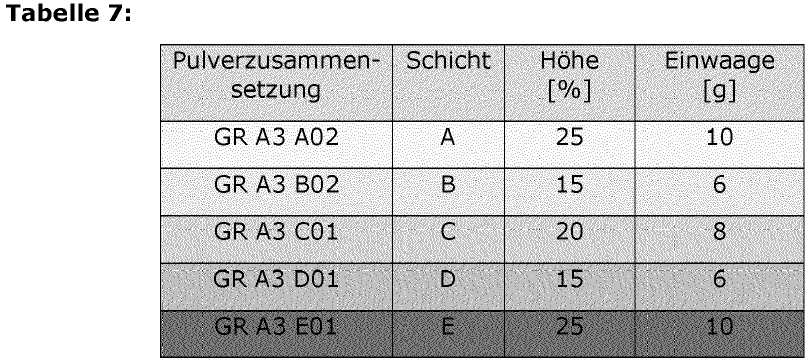

Die Grünlingsdichte der gepressten Rohlinge beträgt 3,08 g/cm3.

Die in den Tabellen 6 und 7 hergestellten Pressrohlinge werden anschließend entbindert. Die Entbinderung erfolgt in einem Thermo-STAR-Ofen. Die Entbinderung bzw. Ansinterung der Blöcke erfolgt mit dem nachfolgenden Entbinderungsprogramm (ca. 20 h). Tmax = 1040°C.

| **Temperatur [°C]** | **Heizrate [°C/min]** | **Haltezeit [min]** |
|---|---|---|
| 30 → 280 | 0,7 | - |
| 280 | - | 90 |
| 280 → 400 | 1 | - |
| 400 → 600 | 10 | - |
| 600 → 1040 | 5 | - |
| 1040 | - | 120 |
| 1040 → 30 | 10 | - |

Die Weißlingsdichte der entbinderten Grünlinge beträgt 3,17 g/cm³.

Die Vickershärte der Weißlinge wurde mit einer Zwick Härteprüfmaschine bestimmt. Dazu wurde die Härte an der Oberseite und der Unterseite jeweils durch 6-fach Messung und Mittelwertbildung bestimmt (Prüfkraft 19,61 N; Belastungsstufe HV 2; Wartezeit am Belastungspunkt: 20 s):
Vickershärte HV 2 (Oberseite): 55, 3
Vickershärte HV 2 (Unterseite): 55,0

Aus dem Weißlingsblock werden 2 Frontzahnkronen gefräst und anschließend endgesintert, wobei die Kronen zunächst auf 1450 °C erwärmt werden und dort für 2 Stunden erhitzt und anschließend kontinuierlich auf Raumtemperatur abgekühlt werden.

Überraschend ist insbesondere die Vickershärte bei den erfindungsgemäßen Sinterkörpern. Während die Vickershärte am vorgesinterten Rohling (Weißling) mit nicht erfindungsgemäßen Sinterkörpern mit zunehmendem Fe2O3-Anteil ansteigt, bleibt die Vickershärte mit ca. 55 HV 2 über den gesamten Block bei der erfindungsgemäßen Sinterkörpern unverändert.

Ästhetisch bietet die Variante mit dem höheren Yttrium-Anteil (Tabelle 8) in der Schneide (Basispulver E) eine etwas höhere Transluzenz entlang der Schneidekante.

## Patentansprüche

1. Gesinterter Formkörper mit Farbverlauf zur Verwendung in der Herstellung dentaler Restaurationen erhältlich durch Sintern eines Pressformkörpers umfassend 2 oder mehr farblich unterschiedliche keramische Pulverschichten, wobei jede Pulverschicht mindestens 80 Gew.-% ZrO₂ aufweist und im Wesentlichen frei von Eisenoxid ist, **dadurch gekennzeichnet, dass** wenigstens eine Pulverschicht Terbiumoxid aufweist.

2. Gesinterter Formkörper gemäß Anspruch 1, **dadurch gekennzeichnet, dass** jede der gesinterten Pulverschichten eine im Wesentlichen gleiche Volumenveränderung über einen Temperaturbereich von 25 bis 1600 °C, vorzugsweise über einen Temperaturbereich von 900 bis 1400 °C, erfährt.

3. Gesinterter Formkörper gemäß Anspruch 1 oder 2, **dadurch gekennzeichnet, dass** jede Pulverschicht eine unterschiedliche Konzentration Terbiumoxid, Erbiumoxid und Cobaltoxid aufweist.

4. Gesinterter Formkörper gemäß einem oder mehreren der vorangehenden Ansprüche, **dadurch gekennzeichnet, dass** der Rohling farblich unterschiedliche Schichten aufweist und durch den Sintervorgang ein gradueller Farbverlauf entsteht.

5. Gesinterter Formkörper gemäß einem oder mehreren der vorangehenden Ansprüche, **dadurch gekennzeichnet, dass** wenigstens 2, vorzugsweise alle Pulverschichten einen im Wesentlichen identischen Yttriumoxid Anteil aufweisen.

6. Gesinterter Formkörper gemäß einem oder mehreren der Ansprüche 1 bis 4, **dadurch gekennzeichnet, dass** der Rohling einen Transluzenzverlauf aufweist, vorzugsweise eine schichtweise Zunahme der Yttriumoxidmenge, aufweist.

7. Gesinterter Formkörper gemäß einem oder mehreren der vorangehenden Ansprüche, **dadurch gekennzeichnet** die Pulverschichten jeweils mindestens 0,02 Gew.-% Al₂O₃, vorzugsweise Al₂O₃ in einer Menge von 0,03 bis 0,15 Gew.-%, insbesondere Al₂O₃ in einer Menge von 0,03 bis 0,08 Gew.-% aufweisen.

8. Gesinterter Formkörper gemäß einem oder mehreren der vorangehenden Ansprüche, **dadurch gekennzeichnet, dass** wenigstens eine, vorzugsweise wenigstens 2 oder wenigstens 3 der Pulverschichten, insbesondere alle Pulverschichten Y₂O₃ und/oder Er₂O₃, vorzugsweise in einer Menge von wenigstens 3 Gew.-%, insbesondere von wenigstens 5 Gew.-% oder wenigstens 6 Gew.-% und insbesondere von 4,5 bis 12 Gew.-%, speziell von 6 bis 10 Gew.-% bezogen auf das Gesamtgewicht der Bestandteile der Pulverschicht, umfasst.

9. Gesinterter Formkörper gemäß einem oder mehreren der vorangehenden Ansprüche **dadurch gekennzeichnet, dass** die Pulverschichten Zirkondioxid und/oder HfO₂ in einer Menge von wenigstens 89 Gew.-%, vorzugsweise in einer Menge von 89 bis 98 Gew.-%, insbesondere von 90 bis 96 Gew.-%, jeweils bezogen auf das Gesamtgewicht der Bestandteile des Basispulvers, aufweist.

10. Gesinterter Formkörper gemäß einem oder mehreren der vorangehenden Ansprüche **dadurch gekennzeichnet, dass** der Pressformkörper aus 4 oder mehr Pulverschichten, vorzugsweise 5 keramischen Pulverschichten besteht.

11. Gesinterter Formkörper gemäß einem oder mehreren der vorangehenden Ansprüche **dadurch gekennzeichnet, dass** der Pressformkörper aus 5 keramischen Pulverschichten besteht, wobei die erste Pulverschicht 20 bis 30 %, vorzugsweise 22 bis 28 %, die zweite Pulverschicht 10 bis 20 %, vorzugsweise 12 bis 18 %, die dritte Pulverschicht 15 bis 25 %, vorzugsweise 17 bis 23 %, die vierte Pulverschicht 10 bis 20 %, vorzugsweise 12 bis 18 % und die fünfte Pulverschicht 20 bis 30 %, vorzugsweise 22 bis 28 %, der Gesamtdicke der übereinander angeordneten Pulverschichten ausmacht und mit der Maßgabe, dass sich die Gesamtdicke auf 100 % ergänzt.

12. Gesinterter Formkörper gemäß einem oder mehreren der vorangehenden Ansprüche **dadurch gekennzeichnet, dass** ein vorgesinterter keramischer Formkörper durch subtraktive Verfahren bearbeitet wird und vorzugsweise anschließend in einem weiteren Schritt endgesintert wird.

13. Gesinterter Formkörper gemäß einem oder mehreren der vorangehenden, **dadurch gekennzeichnet, dass** der Formkörper eine schichtweise Zunahme der Terbiumoxidmenge aufweist.

14. Gesinterter Formkörper gemäß einem oder mehreren der vorangehenden Ansprüche, **dadurch gekennzeichnet, dass** der Formkörper in jeder Pulverschicht Terbiumoxid in einer Menge von 0,001 bis 0,15 Gew.-%, vorzugsweise 0,01 bis 0,1 Gew.-%, insbesondere 0,02 bis 0,06 Gew.-% Terbiumoxid, aufweist.

15. Verwendung des Formkörpers gemäß einem der vorangehenden Ansprüche für dentale Restaurationen oder für die Herstellung dentaler Restaurationen.

16. Verfahren zur Herstellung gesinterten Formkörpers mit Farbverlauf gemäß einem der Ansprüche 1 bis 14 umfassend die folgenden Schritte:
a) Bereitstellen von 2 oder 3 oder 4 oder 5 oder mehr farblich unterschiedlichen keramischen Pulverschichten, die schichtweise übereinander angeordnet sind,
b) Verpressen der schichtweise übereinander angeordneten keramischen Pulverschichten zur Ausbildung eines Pressformkörpers und
c) Sintern des in Schritt b) erhaltenen Formkörpers zur Ausbildung eines keramischen Formkörpers, wobei die keramischen Pulverschichten jeweils eine voneinander verschiedene Zusammensetzung aufweisen und wobei jede Pulverschicht mindestens 80 Gew.-% ZrO₂ aufweist und im Wesentlichen frei von Eisenoxid ist, **dadurch gekennzeichnet, dass** wenigstens eine Pulverschicht Terbiumoxid, speziell Tb₄O₇, aufweist.
